# EUROPEAN PATENT APPLICATION

(11) **EP 2 022 466 A1**
(43) Date of publication of application: **11.02.2009**
(21) Application number: 07015480.2
(22) Date of filing: 07.08.2007
(51) Int. Cl.: A61K 8/03, A61K 8/37, A61Q 5/12

(54) **Two or multi phase composition for conditioning hair**

(71) Applicant: KPSS-Kao Professional Salon Services GmbH, 64297 Darmstadt (DE)
(72) Inventor: Molenda, Michael, 60487 Frankfurt (DE)

(57) **Abstract**

Present invention is related to a composition for conditioning hair comprising two or more optically separated phases when standing at zero shear rate which becomes homogeneous upon shaking. The object of the present invention is a composition for hair comprising 5 to 50%, by weight oil phase, calculated to total composition, and 50 to 95%, by weight, aqueous phase, calculated to total composition, wherein oil phase comprises at least one compound selected from compounds according to general formula 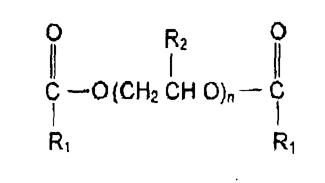 wherein R₁ is a phenyl and R₂ is H or a C1 to C4 alkyl and n is a number between 1 and 5 and compounds of dipropylene glycol dibenzoate and trimethylpentanediyl dibenzoate.

Further object of the invention is the use of,the composition for conditioning hair.

## Description

Present invention is related to a composition for conditioning hair comprising at least two optically separated phases when standing at zero shear rate which becomes homogeneous upon shaking.

Conditioning compositions have been known for many years. Among them it is possible to find products on hair care market for conditioning hair including two separated phases. Such products have been attracting consumers' attention because of their appearance. Since these products are designed mainly for leave in usage, hair is not rinsed off after application of the product, achieving high care level and combining the conditioning benefit for example with additional one or more benefits is found especially attractive.

Two phase compositions on the market deliver good conditioning levels. However there is still need for improvements especially in hair shine and further conditioning effects of such compositions.

WO 02/060397 discloses dual phase conditioning and styling composition for heat styling hair. According to the document compositions comprise an oil phase comprising volatile oil and an aqueous alcoholic phase comprising styling polymer, an emulsifier and a salt.

EP 996 408 discloses dual phased compositions for make up cleansing wherein a polyvinylpyrrolidone copolymer is used as a demixing agent. The documents does not address at all hair care application of such composition.

The inventors of the present invention have surprisingly found out that a composition with at least two phases comprising an oil phase and a water phase wherein two or more phases are optically separated at zero shear rate and becomes homogeneous upon shaking and returns again to optically separated two phases upon release of agitation improves hair shine, elasticity, manageability and combability and adds volume and body when a compound described below is included into the composition.

Accordingly, the object of the present invention is a composition for hair comprising 5 to 50%, by weight oil phase, calculated to total composition, and 50 to 95%, by weight, aqueous phase, calculated to total composition, wherein oil phase comprises at least one compound selected from compounds according to general formula 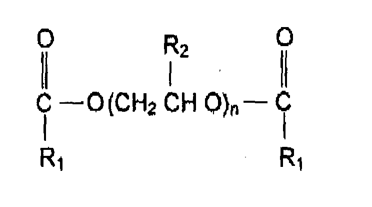 wherein R₁ is a phenyl and R₂ is H or a C1 to C4 alkyl and n is a number between 1 and 5 and compounds of dipropylene glycol dibenzoate and trimethylpentanediyl dibenzoate.

Non-limiting examples to the compounds of the above formula are diethyleneglycol dibenzoate, propylene glycol dibenzoate and triethylene glycol dibenzoate.

Preferred are dipropylene glycol dibenzoate, diethyleneglycol dibenzoate, propylene glycol dibenzoate and triethylene glycol dibenzoate. More preferred are dipropylene glycol dibenzoate, diethyleneglycol dibenzoate and propylene glycol dibenzoate. Most preferred is the compound propylene glycol dibenzoate which is available from inolex Chemical Company under the trade name LexFeel Shine.

Concentration of the compounds mentioned above is in the range of 0.1 to 20%, preferably 0.1 to 15%, more preferably 0.25 to 10% and most preferably 0.5 to 5% by weight calculated to the content of the oil phase.

The invention includes as well compositions with optically separated phases more than two, because it has been observed during the course of testings the inventive compositions especially at high concentration ranges of di benzoate type of compounds the formation of third phase has been observed and this forms at the bottom of the vessel wherein the composition is stored. This is the reason why the compositions are called two of more phase composition. This des not influence the performance on hair.

Compositions of the present invention comprises optically separated two or more phases which becomes homogeneous upon shaking and returns again to optically separated two or more phases upon release of agitation. Compositions of the present invention returns to optically separated two or more phases upon release of agitation preferably within 24 hours, more preferably within 20 hours and most preferably within 10 hours. It should be noted that with the compositions of the present invention separated oil and aqueous phases are recognizable after 15 to 30 minutes of shaking. The above referred separation periods refer to the complete separation of the two or more phases i.e. returning to their original state.

Compositions of the present invention comprises oil phase at a concentration of 5 to 50%, preferably 10 to 40% more preferably 15 to 30% by weight calculated to total composition. Oil phase comprises 70 to 100%, preferably 80 to 100% and more preferably 90 to 100% by weight calculated to the content of oil phase at least one volatile oil. Suitable volatile oils are silicones such as dimethicone and cyclomethicone and mixtures thereof, and volatile hydrocarbons such as isododecane, isohexadecane and isoprafin. The most preferred are silicones and from those dimethicone and cylomethicone and mixtures thereof.

Suitable volatile dimethicones are the ones with a viscosity of less than 5 mm²/s. The most preferred are dimethicone with a viscosity of 1 mm²/s and 0.65 mm²/s which are available from Dow Corning with the trade name DC 200 Fluid.

Suitable volatile cyclomethicones are according to general formula 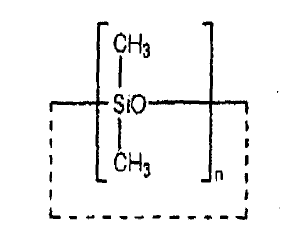 where n is a number between 3 and 7. Preferred are cyclopentasiloxanes known with the trade name for example Dow Corning 245.

Non-volatile silicones may also be incorporated into the compositions of the present invention at a low concentration, i.e. lower than 1% by weight calculated to the content of oil phase. Suitable ones are arylated silicones such as phenyltrimethicone known with the trade name Dow Corning 556, and dimethicones with higher viscosity.

In addition, natural oils such as olive oil, almond oil, avocado oil, wheatgerm oil, ricinus oil can be incorporated into the oil phase. Concentration of natural oils is below 1%, preferably below 0.5% by weight calculated to the content of the oil phase. Solubility of the natural oil in used volatile oil should be examined in selecting natural oil. As a rule selected natural oil should be soluble in the volatile oil used in the composition.

Compositions of the present invention comprise aqueous phase at a concentration of 50 to 95%, preferably 60 to 90% more preferably 70 to 85% by weight calculated to total composition. Aqueous phase comprises 0.1 to 20%, preferably 0.5 to 15%, more preferably 0.5 to 10 % by weight calculated to the content of aqueous phase at least one fixing polymer. The composition can also comprise mixture of more than one fixing polymer. In selecting fixing polymers attention should be paid to the solubility of the polymer in aqueous phase as described above and also in the total composition wherein oil and aqueous phases are present together and mixed to become homogeneous composition by mixing.

Suitable polymers are non-ionic, cationic and amphoteric polymers. Anionic polymers may also be used so far if no incompatibility is observed in the presence of cationic surfactant. One of the important point in selecting fixing polymer is that the polymer should not thicken the aqueous phase. In other words the viscosity of the aqueous phase should remain low i.e. below 100 mPa.s, preferably 50 mPa.s.

Suitable non-ionic polymers are polyvinylpyrrolidone, polyvinylpyrrolidone/vinylacetate copolymer and polyvinylpyrrolidone/vinylacetate/vinylpropionate copolymer. Preferred is polyvinylpyrrolidone/vinylacetate copolymer, In addition to these fixing polymers derivatives of natural polymers such as hydroxyl ethylcellulose may also be used in combination with one of the fixing polymers mentioned above.

Suitable cationic polymers include Polyquaternium-4, Polyquaternium-11, Polyquaternium-16, Polyquaternium-18, Polyquaternium-24, Polyquaternium-28, Polyquaternium-46, polyvinylpyrrolidone/dimethylaminoethyl methacrylate, and cationically derivatized natural polymers. Preferred are Polyquaternium-4, Polyquaternium-11 and Polyquaternium-16.

Suitable amphoteric polymers are those available from the company National Starch under the trade name Amphomer such as Octylacrylamide/Acrylates/Butylaminoethyl methacrylate copolymer, those available under the trade name Diaformer (methacryloylethylbetaine/methaorylates copolymer). Preferred are the ones available under the trade name Amphomer.

In another preferred from of the invention, aqueous phase of the composition comprises combination of non-ionic and cationic fixing polymers.

As a fixing polymer silicone containing polymers either grafted or block copolymer are also useful. Suitable and the preferred one is Polysilicone-9.

Aqueous phase of the compositions of the present invention comprises 5 to 40%, preferably 10 to 35%, more preferably 15 to 30% by weight calculate to the content of aqueous phase at least one water miscible organic solvent-. With the term "water miscible" it is meant that the organic solvent is miscible with water at any ratio. Suitable ones are C₂ - C₄ monohydric alcohols such as ethanol, propanol, isopropanol, butanol and isobutanol, benzyl alcohol and benzyloxyethanol and their mixtures. Most preferred are ethanol and isopropanol.

Aqueous phase of the compositions of the present invention comprises at least one cationic surfactant at a concentration of 0.05 to 2%, preferably 0.1 to 1%, more preferably 0.2 to 0.75% by weight calculated to the content of the aqueous phase. Suitable cationic surfactants are according to the general formula 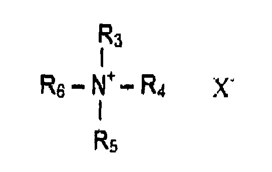 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has typical value of 0 - 4 or

R₈ CO O (CH₂)ₙ

where R₈ is saturated or unsaturated, branched or non-branched alkyl chain with 7-21 C atoms and n has typical value of 0 - 4, and
R₄ is a hydrogen, saturated or unsaturated, branched or non-branched alkyl chain with 1-22 C atoms or

R₇ CO NH (CH₂)ₙ

where R₇ is saturated or unsaturated, branched or non-branched alkyl chain with 7-21 C atoms and n has typical value of 0 - 4 or

R₈ CO O (CH₂)ₙ

where R₈ is saturated or unsaturated, branched or non-branched alkyl chain with 7-21 C atoms and n has typical value of 0 - 4,
and R₅ and R₆ are independent from each other H or lower alkyl chain with 1 to 4 carbon atoms, and X is chloride, bromide or methosulfate.

Suitable cationic surfactants are particularly cetyl trimethly ammonium chloride, steartrimonium chloride, behentrimoinium chloride, stearamidopropyl trimonuim chloride, behenamidopropylethyldimonium ethosulfate, behenamidopropyltrimonium methosulfate, cocamidopropyltrimonium chloride, cocotrimonim chloride, palmitamidopropyltrimonum chloride, dipalmitoyltrimonium chloride, di-C12-C15 alkyldimoniumchloride, distearyldimonium chloride, dipalmitoylethylhydroxyethylmonium chloride, dioleoylethyl dimethyl ammonium methosulfate, dioleoylethyl hydroxyethylmonium methosulfate, dilinolamidopropyldimonium chloride, dioleylethyl hydroxyethylmonium chloride and dipalmitoylethyldimonium chloride.

In another preferred from of the invention the aqueous phase is comprised mixture of two cationic surfactants such as steartrimonium chloride and di-C12-C15 alkyldimoniumchloride.

The pH of the aqueous phase varies from 3 to 7, particularly 4.5 to 6. For adjusting the pH of the said conditioner compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, pyruvic acid, salicylic acid or acetic acid or inorganic acids such as hydrochloric acid, phosphoric acid, sulphuric acid, nitric acid. The pH of the conditioner composition can also be adjusted to the required pH by using alkaline solution such as sodium hydroxide, potassium hydroxide, ammonium hydroxide or their salts with those acids mentioned above.

Further objective of the present invention is the use of the composition comprising 5 to 50%, by weight calculated to total composition, oil phase and 50 to 95%, by weight calculated to total composition, of aqueous phase wherein oil phase comprises at least one volatile oil at a concentration of 70 to 100%, by weight, calculated to the content of oil phase, and at least one compound selected from the compounds according to general formula 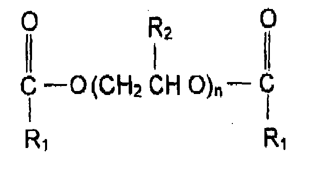 wherein R₁ is a phenyl and R₂ is H or a C1 to C4 alkyl and n is a number between 1 and 5 and/or compound of dipropylene glycol dibenzoate and/or trimethylpentanediyl dibenzoate, and aqueous phase comprises 0.1 to 10% by weight calculated to the content of aqueous phase at least one fixing polymer, 10 to 40% by weight calculate to the content of aqueous phase at least one water miscible organic solvent and 0.05 to 2% by weight calculated to the content of the aqueous phase at least one cationic surfactant wherein oil and aqueous phases are optically separated at zero shear rate and becomes homogeneous upon shaking and returns again to optically separated two phases upon release of agitation for conditioning and shine enhancing of hair..

Compositions of the present invention can comprise UV filters for protection of hair from environmental influences. It should be noted that oil soluble UV filters are contained in the oil phase and water soluble ones are added to the aqueous phase although partitioning the UV filter between two phases is not excluded. The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxybenzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher and/or 2-ethylhexyl-2-cyano-3,3-diphenyl acrylate also known as Octocrylene, and polysiliocne-15

The preferred amount of the UV-absorber ranges from about 0.01 % to 2.5%, more preferably from 0.05 % to 1 % by weight, calculated to the total composition.

The moisturising agents may be included into the aqueous phase of the compositions. Suitable ones are panthenol, polyols, such as glycerol, polyethylene glycols with molecular weight 200 to 20,000. The moisturising ingredients can be included in the aqueous phase of the compositions at a concentration range of 0.01 - 2.5% by weight calculated to the content of the aqueous phase.

Natural plant extracts may as well form part of the compositions of the present invention. Natural extracts should be included into the aqueous phase of the compositions. Suitable aqueous (e.g. steam-distilled) alcoholic or hydro-alcoholic plant extracts known per se are in particular extracts from leaves, fruits, blossoms, roots, rinds or stems of almond, aloe, pineapple, artichoke, arnica, avocado, valerian, henbane, birch, stinging nettle, echinacea, ivy, wild angelica, gentian, ferns, pine needles, silver weed, ginseng, broom, bamboo, green tea, oat, rose hip, hamamelis, hay flowers, elderberry, hop, coltsfoot, currants, chamomile, carrots, chestnuts, clover, burr root, coconut, cocoanut, mango, peach, lemon, cornflower, wheat, apricot, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

The aqueous and oil phases of the compositions of the present invention may be coloured. The dyestuff suitable for product colouring purposes are all useful for this purpose. It should be noted that for colouring aqueous phase water soluble dyes should be used and for colouring oil phase oil soluble dyes are suitable. The mature of the dyestuff is actually not important but preferred are non substantive dyes, i.e, not remaining on hair after washing with water or shampooing.

Fragrance, chelating agent, preservatives and other conventional cosmetic ingredients can be included at their usual concentrations either into the oil or aqueous phases depending on their solubility.

The composition of the present invention is preferably transparent, i.e, both oil and aqueous phases are transparent as judged by a naked eye in a transparent glass or PET bottle with a thickness of up to 3 cm.

Composition of the present invention is leave-in composition, i.e. not rinsed off from hair after application. Accordingly, in a process composition of the present invention is applied to the shampooed and/or wetted hair and hair is dried. It is possible to apply compositions of the present invention as a lotion and also from a bottle equipped with a pump spray. Preferred application is spraying onto hair.

Following examples are to illustrate the invention but not to limit,

### Example 1

| Oil phase | |
|---|---|
| | % by weight |
| Trisiloxane | 94.8 |
| Propylene glycol dibenzoate | 5.0 |
| Fragrance | 0.2 |

| Aqueous phase | |
|---|---|
| | % by weight |
| VP/VA copolymer | 3.5 |
| Steartrimoniumchloride | 0.2 |
| Di-C₁₂-C₁₅ alkyldimonium chloride | 0.1 |
| Ethanol | 23.0 |
| Citric acid/Ammonium hydroxide | q.s. to pH 6.0 |
| Water | to 100 |

The above compositions are transparent compositions. The above composition is used at a concentration of oil phase of 15% by weight and 85% aqueous phase.

It was observed that hair treated with the above composition had excellent shine and elasticity. Additionally hair felt very soft upon touching.

Exclusion of Propylene glycol dibenzoate from the oil phase resulted in significant loss of shine and hair felt slightly less soft.

In the above composition it is observed that a third phase is formed at the bottom of the storage container.

### Example 2

| Oil phase | |
|---|---|
| | % by weight |
| Trisiloxane | 98.6 |
| Propylene glycol dibenzoate | 1.0 |
| Dimethiconol | 1.0 |
| Benzophenone-3 | 0.2 |
| Fragrance | 0.2 |

| Aqueous phase | |
|---|---|
| | % by weight |
| VPNA copolymer | 3.5 |
| Polyqauternium-11 | 0.5 |
| Steartrimoniumchloride | 0.15 |
| Di-C₁₂-C₁₅ alkyldimonium chloride | 0.15 |
| Ethanol | 20.0 |
| Lactic acid/Ammonium hydroxide | q.s. to pH 6.0 |
| Water | to 100 |

The above compositions oil and aqueous phases were mixed in a bottle at a weight ratio of 20% oil phase and 80% aqueous phase. The mixture delivered excellent shine, volume and body to hair.

No third phase formation was observed.

### Example 3

| Oil phase | |
|---|---|
| | % by weight |
| Dimethicone 1 cst | 97.5 |
| Propylene glycol dibenzoate | 2.0 |
| Phenyl trimethicone | 1.0 |
| Octyl methoxy cinnamate | 0.3 |
| Fragrance | 0.2 |

| Aqueous phase | |
|---|---|
| | % by weight |
| VPNA copolymer | 3.0 |
| Polysilicone-9 | 0.5 |
| Cetrimonium chloride | 0.15 |
| Dioleylethyl hydroxyethylmonium chloride | 0.15 |
| Benzophenone-4 | 0.10 |
| Ethanol | 25.0 |
| Lactic acid/Ammonium hydroxide | q.s. to pH 6.0 |
| Dyestuff Cl 19140 and Cl 42053 | q.s. |
| Water | to 100 |

The above compositions were mixed 15% oil phase and 85%by weight aqueous phase. The mixture delivered excellent shine, combability and volume and body to the hair.

Additionally, fly aways were significantly reduced, hair was easily managable and had excellent elasticity

Third pahse formation was observed at the bottom of the container.

### Example 4

| Oil phase | |
|---|---|
| | % by weight |
| Dimethicone 1 cst | 98.6 |
| Propylene glycol dibenzoate | 1.0 |
| Benzophenone-3 | 0.2 |
| Fragrance | 0.2 |

| Aqueous phase | |
|---|---|
| | % by weight |
| VPNA copolymer | 3.0 |
| Polysilicone-9 | 0.5 |
| Cetrimonium chloride | 0.15 |
| Dioleylethyl hydroxyethylmonium chloride | 0.15 |
| Benzophenone-4 | 0.10 |
| Ethanol | 25.0 |
| Lactic acid/Ammonium hydroxide | q.s. to pH 7.0 |
| Dyestuff Cl 19140 and Cl 42053 | q.s. |
| Water | to 100 |

Similar results as observed with example 3 were observed.

## Claims

1. Two or more phase composition for hair **characterised in that** it comprises 5 to 50%, by weight oil phase, calculated to total composition, and 50 to 95%, by weight aqueous phase, calculated to total composition, wherein oil phase comprises at least one compound selected from compounds according to general formula 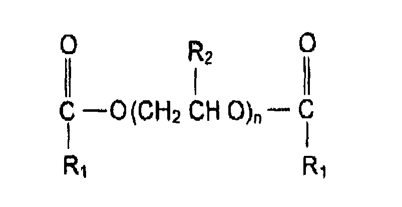 wherein R₁ is a phenyl and R₂ is H or a C1 to C4 alkyl and n is a number between 1 and 5 and compounds of dipropylene glycol dibenzoate and trimethylpentanediyl dibenzoate.

2. Composition according to claim 1 **characterised in that** compounds according to formula are diethyleneglycol dibenzoate, propylene glycol dibenzoate and triethylene glycol dibenzoate.

3. Composition according to claims 1 and 2 **characterised in that** oil phase comprises 70 to 99.9% by weight calculated to content of the oil phase at least one volatile oil.

4. Composition according to any of the preceding claims **characterised in that** aqueous phase comprises 0.1 to 20% by weight calculated to the content of aqueous phase at least one fixing polymer,

5. Composition according to any of the preceding claims **characterised in that** aqueous phase comprises 5 to 40% by weight calculate to the content of aqueous phase at least one water miscible organic solvent.

6. Composition according to any of the preceding claims **characterised in that** aqueous phase comprises at least one cationic surfactant according to general formula 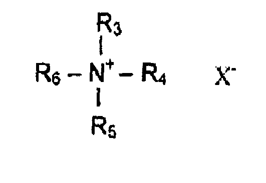 where R₃ is a saturated or unsaturated, branched or non-branched alkyl chain with 8-22 C atoms or
R₇ CO NH (CH₂)ₙ
where R₇ is saturated or unsaturated, branched or non-branched alkyl chain with 7-21 C atoms and n has typical value of 0 - 4 or
R₈ CO O (CH₂)ₙ
where R₈ is saturated or unsaturated, branched or non-branched alkyl chain with 7-21 C atoms and n has typical value of 0 - 4, and
R₄ is a hydrogen, saturated or unsaturated, branched or non-branched alkyl chain with 1-22 C atoms or
R₇ CO NH (CH₂)ₙ
where R₇ is saturated or unsaturated, branched or non-branched alkyl chain with 7-21 C atoms and n has typical value of 0 - 4 or
R₈ CO O (CH₂)ₙ
where R₈ is saturated or unsaturated, branched or non-branched alkyl chain with 7-21 C atoms and n has typical value of 0 - 4,
and R₅ and R₆ are independent from each other H or lower alkyl chain with 1 to 4 carbon atoms, and X is chloride, bromide or methosulfate.

7. Composition according to any of the preceding claims **characterised in that** it comprises at least one UV filter either in oil or in aqueous phase.

8. Composition according to any of the preceding claims **characterised in that** it aqueous phase has a pH between 3 and 7.

9. Composition according to any of the preceding claims **characterised in that** it is transparent.

10. Composition according to any of the preceding claims **characterised in that** it is a 3 phase composition.

11. Use of the composition according to any of the preceding claims for conditioning hair

12. Process for conditioning hair **characterised in that** a composition according to claims 1 to 10 is applied to wet or shampooed and towel dried hair and without rinsing off hair is dried.
